# EUROPEAN PATENT SPECIFICATION

(11) **EP 2 585 041 B1**
(45) Date of publication and mention of the grant of the patent: **31.08.2016**
(21) Application number: 11736275.6
(22) Date of filing: 24.06.2011
(51) Int. Cl.: A61K 9/00, A61K 9/20, A61K 47/10, A61K 47/02

(54) **TASTE-MASKED PHARMACEUTICAL FORMULATION HAVING ACCELERATED ONSET OF ACTION**
GESCHMACKSMASKIERTE FORMULIERUNG MIT RASCHER WIRKUNG
FORMULATION DE MASQUAGE DU GOUT AVEC UN DÉBUT RAPIDE D'ACTION

(30) Priority: 24.06.2010 DE 102010024866
(43) Date of publication of application: 01.05.2013
(73) Proprietor: PHARMATECH GmbH, 24220 Flintbek (DE)
(72) Inventor: STECKEL, Hartwig, 24103 Kiel (DE)
(74) Representative: Samson & Partner Patentanwälte mbB
(86) International application number: PCT/EP2011/003120
(87) International publication number: WO 2011/160849

(56) References cited:
- EP-A2- 0 960 621
- WO-A1-00/57857
- WO-A1-02/05820
- WO-A1-2004/096214
- WO-A1-2008/136636

## Description

### Field of the Invention

The present invention refers to a pharmaceutical composition for oral administration of phosphodiesterase inhibitors and for use for treating male erectile dysfunction.

### Background of the Invention

Due to their bitter or otherwise unpleasant taste, many active ingredients cannot be formulated into a liquid preparation or a preparation for dissolution in the mouth without using taste modifiers or taste masking exipients.

Masking the unpleasant taste of a pharmaceutical agent is one of the primary challenges when developing drugs for peroral or oral administration. In solid dosage forms for peroral administration, taste masking can very often be easily achieved by a functional coating, however, improving the taste of liquid pharmaceutical forms or of pharmaceuticals forms for dissolution in the mouth, is often a major challenge, especially in cases where the unpleasant taste is a bitter taste, which is the most difficult taste to mask of all unpleasant tastes.

Liquid pharmaceutical formulations or pharmaceutical formulations for dissolution within the mouth are often advantageous due to their easy use or their rapid onset of action. In case of the latter pharmaceutical forms, oral dissolution may allow absorption of the active ingredient via the oral mucosa, which enables the active ingredient to reach its target blood concentration faster, and which also enables an accelerated onset of action. Moreover, absorption via the oral mucosa enables a higher initial blood plasma level for active ingredients such as sildenafil citrate which are subject to a first-pass metabolism when absorbed in the gastro-intestinal tract.

EP 0 960 621 A2 discloses pharmaceutical formulations, in particular oral dosage forms, containing sildenafil in the form of its free base, for rapid disintegration.

WO 2004/096214 A1 discloses a rapidly disintegrable composition for masking the bitter taste of ondansetron or a pharmaceutically acceptable salt thereof. In particular, it discloses a composition for oral administration, which rapidly disintegrates in the mouth and masks the bitter taste of ondansetron or a pharmaceutically acceptable salt thereof.

WO 02/05820 A1 discloses orally administrable dosage forms, wherein a solid dispersion of sildenafil citrate, a sugar and, a sugar alcohol selected from mannitol, xylitol, and sorbitol is prepared for taste masking and for increasing the bioavailability and wherein the water solubility of sildenafil citrate is increased.

It is an object of the present invention to develop an oral dosage form, in particular for dissolution within the mouth, of phosphodiesterase inhibitors which have an unpleasant taste and are usually kept in the mouth for as little time as possible.

### Summary of the Invention

The present invention relates to a pharmaceutical composition for oral administration as defined in claim 1.

The present invention relates further to a pharmaceutical composition for use in a method of treating male erectile dysfunction with an accelerated onset of action.

### Detailed Description

First of all, some essential terms will be defined which are used in the description of the present invention.

The term "pharmaceutical compositions for oral administration" or simply "oral pharmaceutical composition" as used herein is defined as a pharmaceutical composition which is deliberately swallowed and/or which is dissolved or chewed and dissolved in the mouth during normal use.

The term "pharmaceutical excipient" is used in its common technical meaning. It refers to all substances other than the active ingredient which are included in a ready-for-use pharmaceutical preparation.

The term "having an unpleasant taste" as used herein is not limited to the fundamental tastes sweet, sour, bitter, umami and salty, but is defined as any taste variant, including sweet, bitter, tangy, alkaline, astringent, hot, dry, tart, cool, warm, burning, sour, spicy, biting, woody, smoky, umami, metallic, and/or as any aftertaste of a composition which is found unpleasant and undesirable.

The term "masking" as used herein is defined as covering, concealing and/or attenuating an unpleasant and undesired taste by adding compounds such as sweeteners, flavoring agents and the like to compositions which contain a compound having an unpleasant or undesired taste, said compound having the unpleasant or undesired taste being left unchanged, its taste, however, being masked by the other taste variants in the composition such that a person who or an animal which takes the composition will not perceive the unpleasant taste, or will at least not find it particularly offending. In the prior art, this type of taste masking is also called "cognitive masking". In the present context, other types of taste masking, such as coating a tablet or micropellets with a coating or film, are referred to as taste neutralization.

It is one aim of the present invention to neutralize or mask the bitter taste of phosphodiesterase inhibitors in order to facilitate their administration in oral preparations, while eliminating the necessity to shorten the exposure of the taste buds of the mouth and of the oral mucosa to the active ingredient as much as possible. Another aim of the present invention is to achieve oral absorption of the active ingredient in the preparation sought after via the oral mucosa.

Therefore, a way had to be found to mask (cognitively mask) the bitter taste of phosphodiesterase inhibitors, since other taste neutralization routes will lead to a reduced exposure of the oral mucosa to the active ingredient, which hinders the absorption of the latter, such other taste neutralization routes including covering, encapsulating or other embedding methods, e.g. by extrusion, spray drying, spray embedding and ion exchanger bonding, or neutralization by decreasing the free molecules concentration of the pharmaceutical agent, as often performed with liquids, e.g. by forming molecule complexes (such as cyclodextrine inclusion compounds), forming ion exchange complexes, using a different counter-ion, forming non-ionic forms of the pharmaceutical agent, or film-coating the particles from suspension, and neutralization by shortening the receptor contact time e.g. by increasing viscosity, using a lipophilic carrier, or forming particulate forms of the phosphodiesterase inhibitor (e.g. in a suspension).

It is known that phosphodiesterase inhibitors are - at least to some extent-absorbed via the oral mucosa.

In the preliminary stages of the present invention, a great number of formulae were prepared and tested which were not able to achieve the desired taste masking effect. For example, we tried to mask the taste of the active ingredient, e.g. sildenafil citrate, via an inclusion compound with cyclodextrines. Furthermore, an excessive addition of sugar alcohols, such as mannitol, sorbitol, xylitol, maltitol, lactitol, erythritol, threitol or Isomalt, and/or of sweeteners alone, such as acesulfame, cyclamate-Na or saccharin-Na, was not successful.

When the excipient used in the present invention comprises sodium hydrogen carbonate, a CO₂-forming substance, in combination with a sufficient amount of sugar alcohols, it is possible to mask the bitter taste of the active ingredient(s) while a separate use of each of the two excipients is not able to achieve this masking effect.

We also observed that the preparation of the invention elicits a rapid onset of action of the phosphodiesterase inhibitors, e.g. sildenafil citrate, an onset that is faster than with peroral administration, which is obviously due to an unexpectedly great amount of active ingredient being absorbed via the oral mucosa.

The inventive composition can, e.g., be formulated as a dry powder, dry granules or a compact (tablet).

The sugar alcohol included in the inventive composition is selected from mannitol, sorbitol, xylitol, maltitol, lactitol, erythritol, threitol, and Isomalt provided that it contains or is Isomalt.

The weight ratio of sugar alcohol, especially Isomalt, to active ingredient may be in the range of up to about 12:1, preferably up to about 7:1, and especially preferred of from about 3:1 up to about 6:1.

Furthermore, the pharmaceutical composition according to the present invention can comprise one or more pharmaceutically acceptable organic acids in order to accelerate the development of CO₂ from sodium hydrogen carbonate. A person skilled in the art knows which acids are pharmaceutically acceptable. They include, among others, citric acid, tartaric acid, succinic acid, fumaric acid, maleic acid, malic acid, and oxalic acid. Citric acid is especially preferred since its own taste can further assist in masking the unpleasant taste of the active ingredient.

Therefore, a preferred composition according to the present invention comprises the following ingredients in the weight proportions stated below:
a) active ingredient in an amount of about 10 parts by weight, more preferred about 25 parts by weight to about 150 parts by weight.
b) sugar alcohol, especially Isomalt, in an amount of about 50 to about 600 part by weight, preferably about 100 to about 300 parts by weight;
c) sodium hydrogen carbonate in an amount of about 2 to about 100 parts by weight, preferably to about 50 parts by weight, and optionally
d) citric acid in an amount of about 2 to about 50 parts by weight, preferably about 5 to about 10 parts by weight or about 10 to about 50 parts by weight.

The phosphodiesterase inhibitor is sildenafil citrate.

Structural formulae of various phosphodiesterase inhibitors are given below. 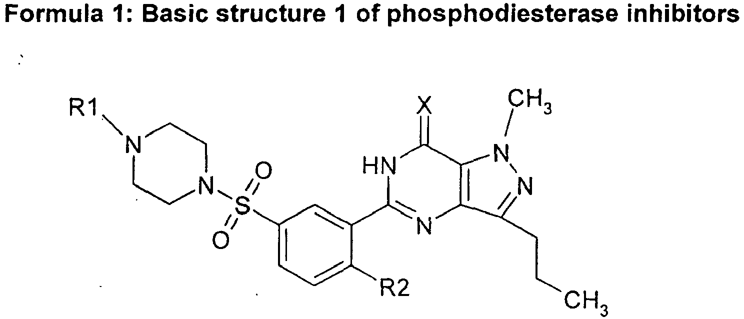 wherein
R1 = methyl, ethyl, propyl, methoxy, ethoxy, hydroxymethyl, hydroxyethyl, H;
R2 = methoxy, ethoxy, propoxy, methyl, ethyl, propyl;
X = O, S, N 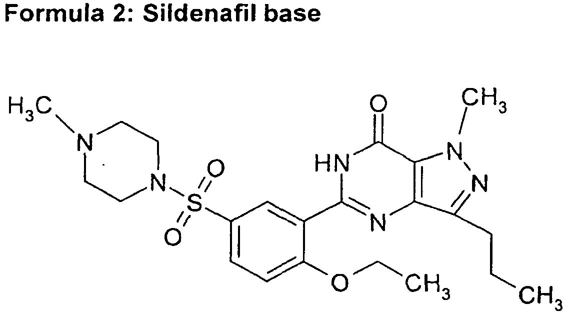 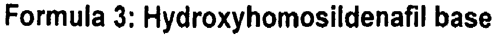 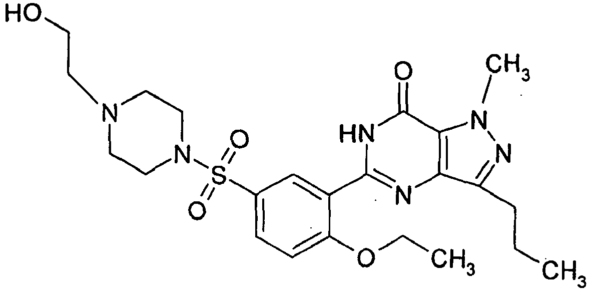 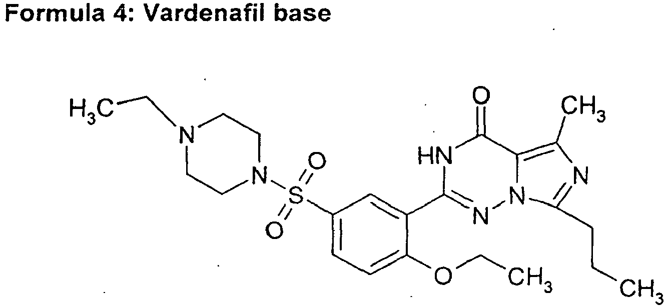 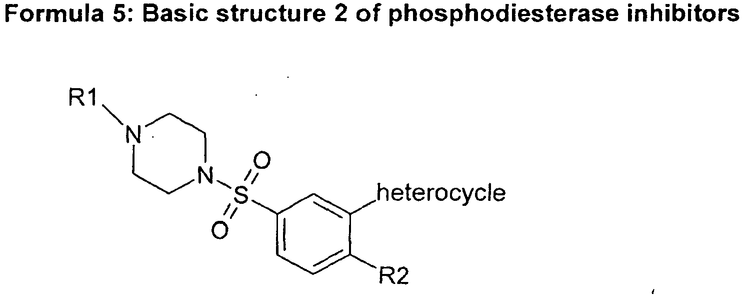 wherein
   R1 = methyl, ethyl, propyl, methoxy, ethoxy, hydroxymethyl, hydroxyethyl, H;
   R2 = methoxy, ethoxy, propoxy, methyl, ethyl, propyl;
   heterocycle = substituted heterocylce

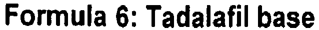

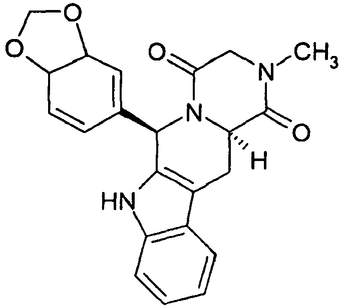

The compositions according to the present invention can for example be administered as a dry powder or as dry granules in a pouch or the like, or as a compact (compacted tablet), which dosage forms are prepared from a powder mixture or granules which comprises at least the active ingredient and the sugar alcohol.

Any of the other excipients which are generally used for the dosage forms mentioned above can be included in the inventive composition. This includes in particular flow additives, e.g. Aerosil^{®} (a colloidal, highly dispersed silica), glidants, e.g. Mg stearate, lubricants, e.g. calcium arachinate, sodium stearyl fumarate or PEG 6000, a disintegrant, e.g. starch, low-substituted hydroxy propyl cellulose or cross-linked colloids, such as Na carboxymethyl cellulose, cross-linked, polyvinyl pyrrolidone, cross-linked, or Na carboxymethyl starch, cross-linked, Polacrilin K, and in particular all types of flavoring agents. Sweeteners may be contained, but this is not preferred.

The preparation of the inventive composition is conventional and comprises e.g. a simple mixing or a granulation of active ingredient and sugar alcohol into a powder mixture or into granules, followed by the addition of sodium hydrogen carbonate and optionally other excipients. Specific preparation methods can be found in the examples.

The onset of action of the phosphodiesterase inhibitors occurs faster than with peroral administration - the absorption via the oral mucosa is independent of food intake, and the bioavailability is not affected by a prior or simultaneous meal.

The following examples serve to further illustrate the invention, without limiting the same.

### Examples

Examples of the inventive compositions are stated below. The figures given in examples 1 to 6 refer to g per dosage unit.

### Example 1 - Formulation example

| | |
|---|---|
| Sildenafil citrate | 0.05 |
| Isomalt | 0.3 |
| citric acid | 0.01 |
| sodium hydrogen carbonate | 0.05 |
| flavors | q.s. |

The substances listed above are classified (355) and processed in a mixer to form a homogeneous powder mixture. To this powder mixture, a flow additive (e.g. Aerosil^{®}), a lubricant (e.g. calcium arachinate, sodium stearyl fumarate or PEG 6000) and/or a disintegrant may be added as needed and depending on the further processing. The powder mixture is filled in a tubular bag ("StickPack").

### Example 2 - Formulation example

| | |
|---|---|
| Sildenafil citrate | 0.025 |
| Isomalt | 0.3 |
| citric acid | 0.01 |
| sodium hydrogen carbonate | 0.05 |
| flavors | q.s. |

The substances listed above are classified (355) and processed in a mixer to form a homogeneous powder mixture. To this powder mixture, a flow additive (e.g. Aerosil^{®}), a glidant (e.g. calcium arachinate, sodium stearyl fumarate or PEG 6000) and/or a disintegrant may be added as needed and depending on the further processing. The powder mixture is compacted to form tablets.

### Example 3 - Formulation example

| | |
|---|---|
| Sildenafil citrate | 0.1 |
| Isomalt | 0.3 |
| citric acid | 0.01 |
| sodium hydrogen carbonate | 0.05 |
| flavors | q.s. |

The substances listed above are classified (355) and processed in a mixer to form a homogeneous powder mixture. To this powder mixture, a flow additive (e.g. Aerosil^{®}), a glidant (e.g. calcium arachinate, sodium stearyl fumarate or PEG 6000) and/or a disintegrant may be added as needed and depending on the further processing. The powder mixture is filled in a tubular bag.

### Example 4 - Formulation example (not according to the invention)

| | |
|---|---|
| Taldalafil | 0.05 |
| Isomalt | 0.3 |
| citric acid | 0.005 |
| sodium hydrogen carbonate | 0.05 |
| flavors | q.s. |

The substances listed above are classified (355) and processed in a mixer to form a homogeneous powder mixture. To this powder mixture, a flow additive (e.g. Aerosil^{®}), a glidant (e.g. calcium arachinate, sodium stearyl fumarate or PEG 6000) and/or a disintegrant may be added as needed and depending on the further processing. The powder mixture is filled in a tubular bag.

### Example 5 - Formulation example (not according to the invention)

| | |
|---|---|
| Hydroxyhomosildenafil | 0.025 |
| Isomalt | 0.3 |
| citric acid | 0.01 |
| sodium hydrogen carbonate | 0.05 |
| flavors | q.s. |

Hydroxyhomosildenafil, Isomalt, and citric acid are granulated with absolute ethanol and passed through a sieve (1000) and dried. The dried granules are mixed with sodium hydrogen carbonate and flavors and processed to form one of the dosage forms mentioned above.

### Example 6 - Activity example

In order to prove the faster oral absorption as compared to a peroral administration of sildenafil citrate, an observational study was performed on 20 subjects who tested the inventive formulation according to example 1 versus Viagra 50 mg with the active ingredient sildenafil citrate, the product commonly marketed in Germany, in a Cross-Over-Design test. The results are summarized in table 1 and prove the unexpectedly considerably shortened time until the onset of action:

| | Time until onset of action [min] | |
|---|---|---|
| Subject No. | Viagra 50 mg | **Inventive Formulation** |
| 1 | 50 | **15** |
| 2 | 60 | **15** |
| 3 | 45 | **10** |
| 4 | 50 | **8** |
| 5 | 60 | **15** |
| 6 | 70 | **12** |
| 7 | 80 | **10** |
| 8 | 55 | **5** |
| 9 | 40 | **10** |
| 10 | 40 | **10** |
| 11 | 40 | **12** |
| 12 | 60 | **8** |
| 13 | 65 | **7** |
| 14 | 75 | **13** |
| 15 | 50 | **10** |
| 16 | 50 | **20** |
| 17 | 45 | **10** |
| 18 | 60 | **8** |
| 19 | 55 | **7** |

Subject 20 only took the "Viagra sample" and left the observation then.

A better masking of the bitter taste versus a comparative formulation which included only Isomalt was confirmed by 5 subjects.

## Claims

1. Pharmaceutical composition for oral administration, comprising a phosphodiesterase inhibitor selected from sildenafil citrate and at least one sugar alcohol selected from mannitol, sorbitol, xylitol, maltitol, lactitol, erythritol, threitol and Isomalt, and sodium hydrogen carbonate as pharmaceutical excipients in the following weight proportions:
phosphodiesterase inhibitor: 10 to 150 parts by weight in total;
sugar alcohol: 50 to 600 parts by weight in total;
sodium hydrogen carbonate: 2 to 100 parts by weight; and
a pharmaceutically acceptable organic acid,
wherein the phosphodiesterase inhibitor and the sugar alcohol are present as a powder mixture or as granules, or as a powder mixture or as granules in a compacted tablet, and
wherein the sugar alcohol includes or is Isomalt.

2. Pharmaceutical composition according to claim 1, **characterized in that** the organic acid comprises citric acid.

3. Pharmaceutical composition according to claim 1 or 2, **characterized in that** the weight ratio of sugar alcohol: active ingredient is up to 7:1 and preferably about 6:1.

4. Pharmaceutical composition according to one of claims 1 to 3, **characterized in that** the pharmaceutical excipients further include a flow additive, such as colloidal, highly dispersed silica, a glidant/lubricant, such as calcium arachinate, sodium stearyl fumarate or PEG 6000, a disintegrant and/or at least one flavoring agent.

5. Pharmaceutical composition according to one of claims 1 to 4 for use in a method of treating male erectile dysfunction with an accelerated onset of action.

## Patentansprüche

1. Pharmazeutische, zur oralen Einnahme bestimmte Zusammensetzung, umfassend einen aus Sildenafilcitrat ausgewählten Phosphodiesterasehemmer und als pharmazeutische Hilfsstoffe mindestens einen aus Mannitol, Sorbitol, Xylitol, Maltitol, Lactitol, Erythritol, Threitol und Isomalt ausgewählten Zuckeralkohol und Natriumhydrogencarbonat in den folgenden Gewichtverhältnissen:
Phosphodiesterasehemmer: 10 bis 150 Gewichtanteile insgesamt;
Zuckeralkohol: 50 bis 600 Gewichtanteile insgesamt;
Natriumhydrogencarbonat: 2 bis 100 Gewichtanteile; und
eine pharmazeutisch annehmbare organische Säure,
wobei der Phosphodiesterasehemmer und der Zuckeralkohol als eine Pulvermischung oder als ein Granulat, oder als eine Pulvermischung oder als Granulat als gepresste Tablette vorhanden sind, und
wobei der Zuckeralkohol Isomalt ist oder enthält.

2. Pharmazeutische Zusammensetzung nach Anspruch 1, **dadurch gekennzeichnet, dass** die organische Säure Citronensäure umfasst.

3. Pharmazeutische Zusammensetzung nach Anspruch 1 oder 2, **dadurch gekennzeichnet, dass** das Gewichtsverhältnis Zuckeralkohol : Wirkstoff bis zu 7:1 und bevorzugt etwa 6:1 beträgt.

4. Pharmazeutische Zusammensetzung nach einem der Ansprüche 1 bis 3, **dadurch gekennzeichnet, dass** die pharmazeutischen Hilfsstoffe ferner ein Fließregulierungsmittel, wie kolloidales, hochdisperses Siliciumdioxid, ein Gleit-/Schmiermittel, wie Calciumarachinat, Natriumstearylfumarat oder PEG 6000, einen Zerfallsbeschleuniger und/oder mindestens ein Aroma enthalten.

5. Pharmazeutische Zusammensetzung nach einem der Ansprüche 1 bis 4 zur Verwendung in einem Verfahren zur Behandlung von erektiler Dysfunktion mit einem schnellen Wirkungseintritt.

## Revendications

1. Composition pharmaceutique pour une administration orale, comprenant un inhibiteur de phosphodiestérase choisi parmi le citrate de sildénafil et au moins un alcool de sucre choisi parmi le mannitol, le sorbitol, le xylitol, le maltitol, le lactitol, l'érythritol, le thréitol et l'isomalt, et de l'hydrogénocarbonate de sodium en tant qu'excipients pharmaceutiques dans les proportions en poids suivantes:
inhibiteur de phosphodiestérase : 10 à 150 parties en poids au total;
alcool de sucre : 50 à 600 parties en poids au total;
hydrogénocarbonate de sodium : 2 à 100 parties en poids; et
un acide organique pharmaceutiquement acceptable,
dans laquelle l'inhibiteur de phosphodiestérase et l'alcool de sucre sont présents sous forme de mélange pulvérulent ou sous forme de granules, ou sous forme de mélange pulvérulent ou sous forme de granules dans un comprimé compacté, et
dans laquelle l'alcool de sucre inclut ou est de l'isomalt.

2. Composition pharmaceutique selon la revendication 1, **caractérisée en ce que** l'acide organique comprend de l'acide citrique.

3. Composition pharmaceutique selon la revendication 1 ou 2, **caractérisée en ce que** le rapport en poids alcool de sucre : ingrédient actif vaut jusqu'à 7 : 1 et de préférence est d'environ 6 : 1.

4. Composition pharmaceutique selon l'une des revendications 1 à 3, **caractérisée en ce que** les excipients pharmaceutiques incluent en outre un additif d'écoulement, tel que de la silice colloïdale hautement dispersée, un agent de glissement/lubrifiant, tel que l'arachinate de calcium, le stéarylfumarate de sodium ou le PEG 6000, un désintégrant et/ou au moins un agent aromatisant.

5. Composition pharmaceutique selon l'une des revendications 1 à 4, pour une utilisation dans une méthode de traitement d'un dysfonctionnement érectile chez l'homme avec un début d'action accéléré.
